(19) Europäisches Patentamt
European Patent Office
Office européen des brevets

(11) Veröffentlichungsnummer : **0 044 266**
**B1**

(12)
# EUROPÄISCHE PATENTSCHRIFT

(45) Veröffentlichungstag der Patentschrift : **18.09.85**

(21) Anmeldenummer : **81710019.1**

(22) Anmeldetag : **29.05.81**

(51) Int. Cl.[4] : **C 07 D271/06**, C 07 D285/08, A 61 K 31/41

(54) **Neue substituierte 3,5-Diamino-1,2,4-oxadiazole und 3,5-Diamino-1,2,4-thiadiazole, Verfahren zu ihrer Herstellung und sie enthaltende pharmazeutische Präparate.**

(30) Priorität : **27.06.80 DE 3024187**

(43) Veröffentlichungstag der Anmeldung : **20.01.82 Patentblatt 82/03**

(45) Bekanntmachung des Hinweises auf die Patenterteilung : **18.09.85 Patentblatt 85/38**

(84) Benannte Vertragsstaaten : **AT BE CH DE FR GB IT LI NL SE**

(56) Entgegenhaltungen :
**DE-A- 2 461 882**
**DE-C- 959 191**
**FR-A- 1 581 394**
**US-A- 2 648 669**
**HETEROCYCLIC CHEMISTRY, Band 5, 1965, Academic Press New York, U.S.A., ausg. durch A.R. KATRITZKI**
**Helv. Chim. Acta 63, 841,859 (1980); Ibid 63, 832-840 (1980), J. Chem. Soc. (1956), 2345-52**
**Die Akte enthält technische Angaben, die nach dem Eingang der Anmeldung eingereicht wurden und die nicht in dieser Patentschrift enthalten sind.**

(73) Patentinhaber : **Beiersdorf Aktiengesellschaft**
**Unnastrasse 48**
**D-2000 Hamburg 20 (DE)**

(72) Erfinder : **Cohnen, Erich, Dr. Dipl.-Chem.**
**Heilwigstrasse 25**
**D-2000 Hamburg 20 (DE)**
Erfinder : **Armah, Ben, Dr.**
**Milcher Strasse 9d**
**D-2000 Hamburg 52 (DE)**

**EP 0 044 266 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind neue substituierte 3,5-Diamino-1,2,4-oxadiazole und 3,5-Diamino-1,2,4-thiadiazole der allgemeinen Formel I

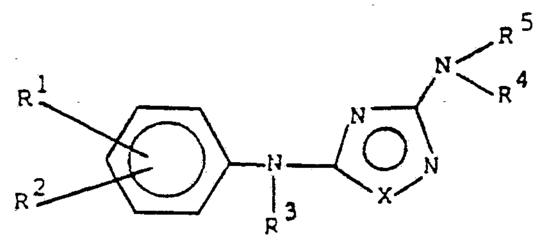

(I)

in der

$R^1$ ein Wasserstoff- oder Halogenatom oder eine Alkylgruppe,

$R^2$ ein Halogenatom oder eine Alkylgruppe,

$R^3$ ein Wasserstoffatom, eine Acylgruppe oder eine gesättigte oder ungesättigte Alkylgruppe,

$R^4$ und $R^5$, die gleich oder verschieden sein können, ein Wasserstoffatom oder eine Alkylgruppe bedeuten, wobei sämtliche Alkyl- oder Acylgruppen verzweigt oder unverzweigt sein können und jeweils 1 bis 4 Kohlenstoffatome enthalten, und

X für Sauerstoff oder Schwefel steht, ausgenommen

3-Amino-5-p-bromanilino-1,2,4-thiadiazol, 3-Amino-5-(p-tolylamino)-1,2,4-thiadiazol und 3-Amino-5-(4-methyl-phenylamino)-1,2,4-oxadiazol.

Verfahren zu ihrer Herstellung und diese Verbindungen enthaltenden Arzneimittel.

Einige Verbindungen sind bereits in der Literatur (Helvetia Chimica Acta *63* (1980), 832 und 841 sowie F. Kurzer, Journal of the Chemical Society (1956), 2345-52) beschrieben und vorstehend im einzelnen genannt. Die erfindungsgemäßen Verbindungen zeichnen sich in überraschender Weise durch eine wesentlich höhere Wirkungsstärke aus.

Bevorzugte Acylgruppen sind Alkylcarbonylgruppen mit 1 bis 3 Kohlenstoffatomen im Alkylteil und die Formylgruppe. Die Alkylgruppe kann geradkettig oder verzweigt sein.

Bevorzugt werden Verbindungen der allgemeinen Formel I in der $R^1$ und $R^2$ Methylgruppen oder Fluor-, Chlor- oder Bromatome, insbesondere aber Chloratome bedeuten. Vorzugsweise befinden sich Substituenten in 2,6- und 2,3-Stellung. $R^3$ bedeutet vorzugsweise ein Wasserstoffatom oder die Acetylgruppe. Bevorzugte Reste $R^4$ und $R^5$ sind Wasserstoffatome oder Methyl- und Äthylgruppen.

Besonders bevorzugt werden Verbindungen der allgemeinen Formel I, in der die Gruppierung —X— für —O— steht und der 5-Phenylamino-Ring mit $R^1$ und $R^2$ zweifach substituiert ist, wobei $R^1$ und $R^2$ Methyl- und Äthylgruppen und/oder Halogenatome, vorzugsweise Brom und/oder Chloratome, vorzugsweise in der 2,3-Stellung, insbesondere aber in der 2,6-Stellung, bedeuten.

Weiterhin werden 3,5-Diamino-1,2,4-oxadiazole der allgemeinen Formel I bevorzugt, in der die Gruppierung —X— für —O— steht und $R^1$, $R^2$, $R^3$, $R^4$ und $R^5$ die angegebene Bedeutung haben, wobei, wenn $R^1$ eine 4-Methylgruppe bedeutet, mindestens einer der restlichen Substituenten nicht Wasserstoff ist.

Ebenfalls werden Verbindungen der allgemeinen Formel I bevorzugt, in der die Gruppierung —X— für —S— steht und $R^1$ und/oder $R^2$ Methyl- oder Äthylgruppen, Halogenatome, vorzugsweise Brom und/oder Chloratome, in der 2,3-Stellung, insbesondere aber in der 2,6-Stellung, bedeuten.

Die erfindungsgemäßen neuen Verbindungen und deren Säureadditionssalze besitzen wertvolle therapeutische Eigenschaften. Sie zeichnen sich durch eine starke bradycarde Wirkung und insbesondere durch eine starke und langanhaltende antihypertensive Wirkung aus und können daher zur Behandlung der Hypertonie verwendet werden. Die Verbindungen können oral oder parenteral angewendet werden. Eine Dosierung von 1-10 mg/kg p. o. ruft bei renal oder genetisch hypertensiven Ratten eine langanhaltende Blutdrucksenkung hervor. Die Dosierung beim Menschen beträgt 0,5-30 mg pro Tag.

In den Beispielen und nachstehend sind bevorzugte Verbindungen der allgemeinen Formel I mit einem therapeutischen Effekt aufgeführt :

3-Amino-5-(2-chlor-3-brom-phenylamino)-1,2,4-oxadiazol
3-Amino-5-(2-brom-3-chlor-phenylamino)-1,2,4-oxadiazol
3-Amino-5-(2-chlor-4-methyl-phenylamino)-1,2,4-oxadiazol
3-Amino-5-(2-chlor-6-methyl-phenylamino)-1,2,4-oxadiazol
3-Amino-5-(2-chlor-3-methyl-phenylamino)-1,2,4-oxadiazol
3-Amino-5-(2,6-dibrom-phenylamino)-1,2,4-oxadiazol
3-Amino-5-(2,4-dimethyl-phenylamino)-1,2,4-oxadiazol
3-Methylamino-5-(2,6-dichlor-phenylamino)-1,2,4-oxadiazol
3-Dimethylamino-5-(2,6-dichlorphenylamino)-1,2,4-oxadiazol
3-Amino-5-(2-chlor-4-methyl-phenylamino)-1,2,4-thiadiazol

3-Amino-5-(2-chlor-6-methyl-phenylamino)-1,2,4-thiadiazol
3-Dimethylamino-5-(2,6-dichlorphenylamino)-1,2,4-thiadiazol
3-Dibutylamino-5-(2,6-dichlorphenylamino)-1,2,4-thiadiazol
3-Dibutylamino-5-(2,6-dichlorphenylamino)-1,2,4-oxadiazol
3-Propylamino-5-(2,6-dichlorphenylamino)-1,2,4-oxadiazol.

Eine besonders bevorzugte Verbindung mit einem hohen therapeutischen Effekt ist 3-Amino-5-(2,6-dichlorphenyl-amino)-1,2,4-oxadiazol.

Gemäß der Erfindung werden pharmazeutische Zusammensetzungen geschaffen, die eine Verbindung der Formel I zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können ein oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, z. B. inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, z. B. Suspendiermitteln wie Methylcellulose, Tragacanth oder Natriumalginat, Netzmitteln wie Lecithin, Polyoxyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90 %, insbesondere 1 bis 90 %, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 0,25 bis 30 mg zur Behandlung der Hypertonie.

Die Verbindungen der allgemeinen Formel I sind nach folgenden Verfahren erhältlich :

a) Das Verfahren zur Herstellung der 1,2,4-Oxadiazole der allgemeinen Formel I, in der X für ein Sauerstoffatom steht, $R^1$, $R^2$, $R^4$ und $R^5$ die oben angegebene Bedeutung haben und $R^3$ ein Wasserstoffatom bedeutet, ist dadurch gekennzeichnet, daß man substituierte Isocyaniddichloride der allgemeinen Formel II

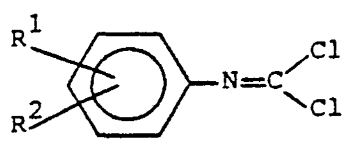
(II)

in der $R^1$ und $R^2$ die oben angegebene Bedeutung haben, mit einem Hydroxyguanidin der allgemeinen Formel III

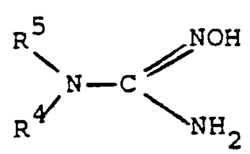
(III)

in der $R^4$ und $R^5$ die oben angegebene Bedeutung haben, in Gegenwart einer Base zu den erfindungsgemäßen 3,5-Diamino-1,2,4-oxadiazolen cyclisiert.

Bevorzugte Basen sind Natrium- oder Calciumhydroxid, wobei als Lösungsmittel Dioxan-Wasser-Gemische verwendet werden. Die substituierten Hydroxyguanidine können auch in der Form ihrer Säureadditionssalze eingesetzt werden.

Die als Ausgangsverbindungen verwendeten phenylsubstituierten Isocyaniddichloride der allgemeinen Formel II und die gegebenenfalls alkylsubstituierten Hydroxyguanidine der allgemeinen Formel III sind bekannt oder nach bekannten Verfahren erhältlich.

b) Die 1,2,4-Thiadiazole der allgemeinen Formel I, in der X ein Schwefelatom bedeutet, $R^1$, $R^2$, $R^4$

und $R^5$ die angegebene Bedeutung haben und $R^3$ ein Wasserstoffatom darstellt, können durch oxidative Cyclisierung von phenylsubstituierten Amidino-thioharnstoffen der allgemeinen Formel IV

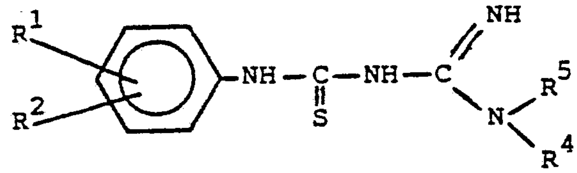

(IV)

in der $R^1$, $R^2$, $R^4$ und $R^5$ die oben angegebene Bedeutung haben, hergestellt werden.

Geeignete Oxidationsmittel sind N-Halogensuccinimide, tert.-Butylhypochlorit, Alkalihypohalogenite oder Wasserstoffperoxid. Die Reaktion wird bei Temperaturen zwischen 20 und 100 °C durchgeführt.

Die als Ausgangsmaterialien benutzten Amidino-thioharnstoffe der allgemeinen Formel IV werden durch an sich bekannte Umsetzung von entsprechenden phenylsubstituierten Isothiocyanaten mit entsprechend alkylsubstituierten Guanidinen bzw. Guanidin gewonnen. Die hierfür als Ausgangsverbindungen verwendenten Isothiocyanate und Guanidine sind bekannte oder nach bekannten Verfahren erhältlich.

c) 1,2,4-Oxadiazole und 1,2,4-Thiadiazole der allgemeinen Formel I, in der $R^3$ eine Acylgruppe bedeutet und $R^1$, $R^2$, $R^4$ und $R^5$ die angegebene Bedeutung haben, sind zugänglich durch Umsetzung von Verbindungen der Formel I, in denen $R^3$ ein Wasserstoffatom bedeutet, mit Acylhalogeniden oder Säureanhydriden in Gegenwart von organischen Basen, bevorzugt Pyridin.

d) 1,2,4-Oxadiazole und 1,2,4-Thiadiazole der allgemeinen Formel I, in der $R^3$ eine Alkylgruppe oder Alkenylgruppe und $R^1$, $R^2$, $R^4$ und $R^5$ die angegebene Bedeutung haben, erhält man durch Umsetzung von Verbindungen der Formel I, in denen $R^3$ ein Wasserstoffatom bedeutet, mit Alkylhalogeniden oder Alkenylhalogeniden und Basen. Bevorzugte Basen sind Alkalialkoholate, wobei die entsprechenden Alkohole als Lösungsmittel Verwendung finden.

Die folgenden Beispiele zeigen spezielle Ausführungsformen und dienen zur Erläuterung vorliegender Erfindung :

## Beispiel 1

3-Amino-5-(2,6-dichlorphenylamino)-1,2,4-oxadiazol

39,2 g (0,16 Mol) 2,6-Dichlorphenyl-isocyaniddichlorid in 270 ml Dioxan werden bei 20 °C zu einer Lösung von 20 g (0,16 Mol) Hydroxyguanidin-sulfat in 240 ml 2 N Natronlauge getropft. Nach 15 Stunden wird das Dioxan im Vakuum abdestilliert und die wässrige Phase mit Methylenchlorid extrahiert. Nach Säulenchromatographie an Kieselgel erhält man 4,5 g 3-Amino-5-(2,6-dichlorphenylamino)-1,2,4-oxadiazol.

Fp. 180-181 °C.

Analog Beispiel 1 wurden folgende in der folgenden Tabelle aufgeführten Verbindungen der allgemeinen Formel I dargestellt :

| Beispiel Nr. | $R^1$ | $R^2$ | $R^3$ | $R^4$ | $R^5$ | X | Fp °C |
|---|---|---|---|---|---|---|---|
| 2 | 2-$CH_3$ | 6-$CH_3$ | H | H | H | O | 151-152 |
| 3 | 3-Cl | 4-Cl | H | H | H | O | 153-154 (Z.) |
| 4 | 2-$CH_3$ | 5-F | H | H | H | O | 162-164 |
| 5 | 2-$CH_3$ | 4-Cl | H | H | H | O | 186-188 (Z.) |
| 6 | 2-Cl | 3-Cl | H | H | H | O | 148-149 |
| 7 | 2-Br | 3-Br | H | H | H | O | 187-188 |

## Beispiel 8

3-Amino-5-(2,6-dimethylphenylamino)-1,2,4-thiadiazol

Eine Lösung von 7,0 g (0,03 Mol) 1-Amidino-3-(2,6-dimethylphenyl)-thioharnstoff in 150 ml Methylenchlorid wird mit 4,2 g (0,03 Mol) N-Chlor-succinimid (95 %ig) 1 Stunde bei 20 °C gerührt, dann noch

eine weitere Stunde nach Zusatz von 15 ml konz. NaOH. Man verdünnt mit Wasser, trennt nach Filtration die Methylenchlorid-Phase ab und erhält nach Säulenchromatographie an Kieselgel ($MeOH/CHCl_3$) : 10/90) 4,8 g 3-Amino-5-(2,6-dimethylphenylamino)-1,2,4-thiadiazol.

Fp. 180-182 °C.

Beispiel 9

3-Amino-5-(2,6-dichlorphenylamino)-1,2,4-thiadiazol

Zu einer Lösung von 4 g 1-Amidino-3-(2,6-dichlorphenyl)-thioharnstoff in 35 ml Äthanol werden nach Zusatz von 1,7 ml konz. Salzsäure bei Siedetemperatur 26 ml 6 %iges $H_2O_2$ getropft. Nach Abdampfen des Äthanols wird der Rückstand in Natriumhydrogencarbonatlösung suspendiert, filtriert und mit Wasser gewaschen. Man erhält 2,8 g 3-Amino-5-(2,6-dichlorphenylamino)-1,2,4-thiadiazol.

Fp. 178-179 °C.

Analog Beispiel 8 und 9 wurden folgende Verbindungen synthetisiert :

Beispiel 10

3-Amino-5-(2,3-dichlorphenylamino)-1,2,4-thiadiazol.

Fp. 161-163 °C.

Beispiel 11

3-Amino-5-(2,6-dibromphenylamino)-1,2,4-thiadiazol.

Fp. 224-227 °C.

Die zur Herstellung der 1,2,4-Thiadiazole verwendeten Amidinothioharnstoffe sind neue Verbindungen und können wie in Beispiel 12 beschrieben synthetisiert werden.

Beispiel 12

1-Amidino-3-(2,6-dichlorphenyl)-thioharnstoff

12 g (0,06 Mol) 2,6-Dichlorphenylisothiocyanat in 100 ml Dimethoxyäthan werden mit 5,4 g (0,09 Mol) Guanidin versetzt und 1 Stunde bei 25 °C gerührt. Nach Abdampfen des Lösungsmittels wird der Rückstand aus Äthanol umkristallisiert.

Ausbeute : 6,4 g

Fp. 189-190 °C

Analog Beispiel 12 wurden folgende Verbindungen hergestellt :

1-Amidino-3-(2,6-dimethylphenyl)-thioharnstoff

Fp. 154-156 °C

1-Amidino-3-(2,3-dichlorphenyl)-thioharnstoff

Fp. 150-152 °C

1-Amidino-3-(2,6-dibromphenyl)-thioharnstoff

Fp. 220-222 °C

Beispiel 13

3-Amino-5-[N-acetyl-N-(2,6-dichlorphenyl)-amino]-1,2,4-oxadiazol

2,4 g (0,01 Mol) 3-Amino-5-(2,6-dichlorphenylamino)-1,2,4-oxadiazol in 10 ml Pyridin werden bei Raumtemperatur mit 5 ml Acetanhydrid versetzt und 2 Stunden bei 25-30 °C gerührt. Nach Abdampfen des Lösungsmittels wird der Rückstand in $CHCl_3$ aufgenommen und mit Natriumhydrogencarbonat-Lösung gegengeschüttelt. Die $CHCl_3$-Phase enthält 3 g der N-Acetyl-Verbindung, die durch Säulenchromatographie an Kieselgel gereinigt wird.

Fp. 164-165 °C.

Beispiel 14

3-Amino-5-[N-(2,6-dichlorphenyl)-N-methyl-amino]-1,2,4-oxadiazol

3,0 g (0,012 Mol) 3-Amino-5-(2,6-dichlorphenylamino)-1,2,4-oxadiazol werden einer Lösung von 0,6 g (0,025 Mol) Natrium in 100 ml Methanol hinzugefügt und 1,6 ml (0,024 Mol) Methyljodid zugetropftund 24 Stunden bei Raumtemperatur gerührt. Man zieht das Lösungsmittel im Vakuum ab, nimmt den Rückstand in Methylenchlorid auf, extrahiert mit Wasser und erhält nach Abdampfen des Lösungsmittels 2,8 g der N-Methyl-Verbindung, die nach Umkristallisation aus Essigester/Diisopropyläther einen Fp. von 177-178 °C aufweist.

Analog Beispiel 14 wurden folgende Verbindungen aus den entsprechenden Verbindungen, in denen $R^3$ ein Wasserstoffatom darstellt, erhalten :

| Beispiel | $R^1$ | $R^2$ | $R^3$ | $R^4$ | $R^5$ | X | Fp °C |
|---|---|---|---|---|---|---|---|
| 15 | 2-Cl | 6-Cl | $(CH_3)_2CH-$ | H | H | O | 175-177 |
| 16 | 2-Cl | 6-Cl | $CH_3(CH_2)_3-$ | H | H | O | 126-127 |
| 17 | 2-Cl | 6-Cl | $-CH_2CH=CH_2$ | H | H | O | 143-144 |
| 18 | 2-Cl | 6-Cl | $-CH_3$ | H | H | S | 181-182 |

Beispiel 15

Herstellung von Tabletten

Tabletten, die die im folgenden genannten Bestandteile enthalten, lassen sich in bekannter Weise herstellen. Sie können zur Behandlung der Hypertonie in einer Dosis von 1 bis 2 Tabletten zweimal täglich verwendet werden.

| | |
|---|---|
| 3-Amino-5-(2,6-dichlorphenylamino)-1,2,4-oxadiazol | 0,25 mg |
| Lactose | 75 mg |
| Maisstärke | 10 mg |
| Mikrokristalline Cellulose | 8 mg |
| Polyvinylpyrrolidon | 1 mg |
| Magnesiumstearat | 0,5 mg |
| Hochdisperses Siliziumdioxid | 0,5 mg |

**Patentansprüche** (für die Vertragsstaaten : BE, CH, DE, FR, GB, IT, LI, NL, SE)

1. Substituierte 3,5-Diamino-1,2,4-oxadiazole und 3,5-Diamino-1,2,4-thiadiazole der allgemeinen Formel I

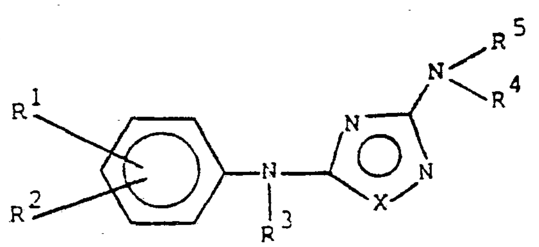

(I)

in der

$R^1$ ein Wasserstoff- oder Halogenatom oder eine Alkylgruppe,

$R^2$ ein Halogenatom oder eine Alkylgruppe,

$R^3$ ein Wasserstoffatom, eine Acylgruppe oder eine gesättigte oder ungesättigte Alkylgruppe,

$R^4$ und $R^5$ die gleich oder verschieden sein können, ein Wasserstoffatom oder eine Alkylgruppe bedeuten, wobei sämtliche Alkyl- oder Acylgruppen verzweigt oder unverzweigt sein können und jeweils 1 bis 4 Kohlenstoffatome enthalten und

X Sauerstoff oder Schwefel bedeutet, ausgenommen

3-Amino-5-(4-methyl-phenylamino)-1,2,4-oxadiazol

3-Amino-5-p-bromanilino-1,2,4-thiadiazol

3-Amino-5-(p-tolylamino)-1,2,4-thiadiazol.

2. 3,5-Diamino-1,2,4-oxadiazole der allgemeinen Formel I gemäß Anspruch 1, in der —X— für —O— steht und $R^1$, $R^2$, $R^3$, $R^4$ und $R^5$ die angegebene Bedeutung haben, wobei, wenn $R^1$ die 4-Methylgruppe bedeutet, mindestens einer der restlichen Substituenten $R^2$, $R^3$, $R^4$ und $R^5$ nicht Wasserstoff ist.

3. 3-Amino-5-(2,6-dichlorphenylamino)-1,2,4-oxadiazol gemäß Anspruch 1.

4. 3,5-Diamino-1,2,4-thiadiazole gemäß Anspruch 1.

5. Verfahren zur Herstellung der 1,2,4-Oxadiazole gemäß der allgemeinen Formel I gemäß Anspruch 1, in der X für ein Sauerstoffatom steht, $R^1$, $R^2$, $R^4$ und $R^5$ die oben angegebene Bedeutung haben und $R^3$ ein Wasserstoffatom bedeutet, dadurch gekennzeichnet, daß man Isocyaniddichloride der allgemeinen Formel II

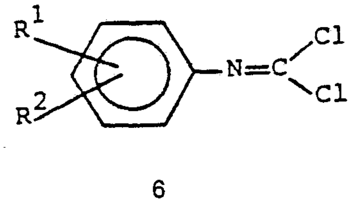

(II)

in der $R^1$ und $R^2$ die oben angegebene Bedeutung haben, mit einem Hydroxyguanidin der allgemeinen Formel III

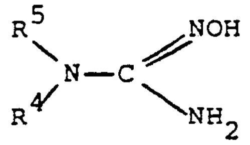

(III)

in der $R^4$ und $R^5$ die oben genannte Bedeutung haben, in Gegenwart einer Base zu den erfindungsgemäßen 3,5-Diamino-1,2,4-oxadiazolen cyclisiert.

6. Verfahren zur Herstellung der 1,2,4-Thiadiazole gemäß der allgemeinen Formel I gemäß Anspruch 1, in der X für ein Schwefelatom steht und $R^1$, $R^2$, $R^4$ und $R^5$ die oben angegebene Bedeutung haben und $R^3$ ein Wasserstoffatom ist, dadurch gekennzeichnet, daß man Amidinothioharnstoffe der allgemeinen Formel IV

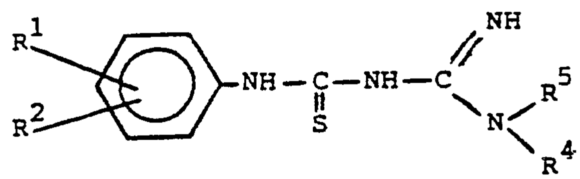

(IV)

in der $R^1$, $R^2$, $R^4$ und $R^5$ die oben angegebene Bedeutung haben, oxidativ zu den 3,5-Diamino-1,2,4-thiadiazolen cyclisiert.

7. Verfahren zur Herstellung von Verbindungen der allgemeinen Formel I gemäß Anspruch 1, in der $R^3$ eine Acylgruppe bedeutet und $R^1$, $R^2$, $R^4$, $R^5$ und X die angegebene Bedeutung haben, dadurch gekennzeichnet, daß man Verbindungen der allgemeinen Formel I, in der $R^3$ ein Wasserstoffatom ist, mit Acylhalogeniden oder Säureanhydriden umsetzt.

8. Verfahren zur Herstellung von Verbindungen der allgemeinen Formel I gemäß Anspruch 1, in der $R^3$ eine Alkylgruppe bedeutet und $R^1$, $R^2$, $R^4$, $R^5$ und X die angegebene Bedeutung haben, dadurch gekennzeichnet, daß man Verbindungen der allgemeinen Formel I, in der $R^3$ ein Wasserstoffatom ist, mit Alkylhalogeniden umsetzt.

9. Pharmazeutisches Präparat, dadurch gekennzeichnet, daß es eine oder mehrere der Verbindungen der allgemeinen Formel I gemäß Anspruch 1 oder deren Verträgliche Salze und gegebenenfalls übliche Trägerstoffe und/oder Verdünnungsmittel enthält.

10. Verbindungen der allgemeinen Formel I gemäß Anspruch 1 zur Anwendung bei Hypertonie.

**Patentansprüche** (für den Vertragsstaat AT)

1. Verfahren zur Herstellung von substituierten 3,5-Diamino-1,2,4-oxadiazolen und 3,5-Diamino-1,2,4-thiadiazolen der allgemeinen Formel I

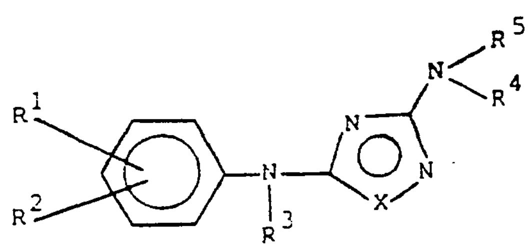

(I)

in der

$R^1$ ein Wasserstoff- oder Halogenatom oder eine Alkylgruppe,

$R^2$ ein Halogenatom oder eine Alkylgruppe,

$R^3$ ein Wasserstoffatom, eine Acylgruppe oder eine gesättigte oder ungesättigte Alkylgruppe,

$R^4$ und $R^5$ die gleich oder verschieden sein können, ein Wasserstoffatom oder eine Alkylgruppe bedeuten, wobei sämtliche Alkyl- oder Acylgruppen verzweigt oder unverzweigt sein können und jeweils 1 bis 4 Kohlenstoffatome enthalten und

X Sauerstoff oder Schwefel bedeutet, ausgenommen 3-Amino-5-(4-methyl-phenylamino)-1,2,4-oxadiazol 3-Amino-5-p-bromanilino-1,2,4-thiadiazol 3-Amino-5-(p-tolylamino)-1,2,4-thiadiazol,

dadurch gekennzeichnet, daß man

a) zur Herstellung der 1,2,4-Oxadiazole der allgemeinen Formel I, in der X für ein Sauerstoffatom

steht, $R^1$, $R^2$, $R^4$ und $R^5$ die oben angegebene Bedeutung haben und $R^3$ ein Wasserstoffatom bedeutet, Isocyaniddichloride der allgemeinen Formel II

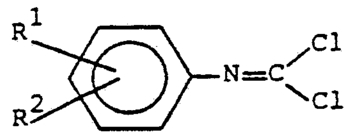

(II)

in der $R^1$ und $R^2$ die oben angegebene Bedeutung haben, mit einem Hydroxyguanidin der allgemeinen Formel III

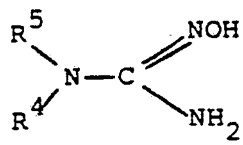

(III)

in der $R^4$ und $R^5$ die obengenannte Bedeutung haben, in Gegenwart einer Base zu den erfindungsgemäßen 3,5-Diamino-1,2,4-oxadiazolen cyclisiert,

b) zur Herstellung der 1,2,4-Thiadiazole gemäß der allgemeinen Formel I, in der X für ein Schwefelatom steht und $R^1$, $R^2$, $R^4$ und $R^5$ die oben angegebene Bedeutung haben und $R^3$ ein Wasserstoffatom ist, Amidinothioharnstoffe der allgemeinen Formel IV

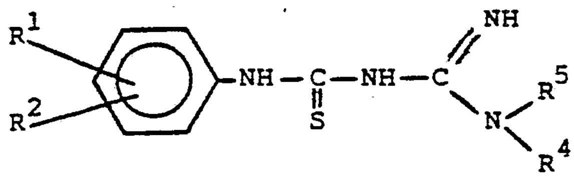

(IV)

in der $R^1$, $R^2$, $R^4$ und $R^5$ die oben angegebene Bedeutung haben, oxidativ zu den 3,5-Diamino-1,2,4-thiadiazolen cyclisiert,

c) zur Herstellung von Verbindungen der allgemeinen Formel I, in der $R^3$ eine Acylgruppe bedeutet und $R^1$, $R^2$, $R^4$, $R^5$ und X die angegebene Bedeutung haben, Verbindungen der allgemeinen Formel I, in der $R^3$ ein Wasserstoffatom ist, mit Acylhalogeniden oder Säureanhydriden umsetzt und

d) zur Herstellung von Verbindungen der allgemeinen Formel I, in der $R^3$ eine Alkylgruppe bedeutet und $R^1$, $R^2$, $R^4$, $R^5$ und X die angegebene Bedeutung haben, Verbindungen der allgemeinen Formel I, in der $R^3$ ein Wasserstoffatom ist, mit Alkylhalogeniden umsetzt.

2. Verfahren nach Anspruch 1 zur Herstellung von 3-Amino-5-(2,6-dichlorphenylamino)-1,2,4-oxadiazol, dadurch gekennzeichnet, daß man 2,6-Dichlorphenylisocyaniddichlorid mit Hydroxyguanidin cyclisiert.

**Claims** (for the contracting states : BE, CH, DE, FR, GB, IT, LI, NL, SE)

1. Substituted 3,5-diamino-1,2,4-oxadiazoles and 3,5-diamino-1,2,4-thiadiazoles of the general formula I

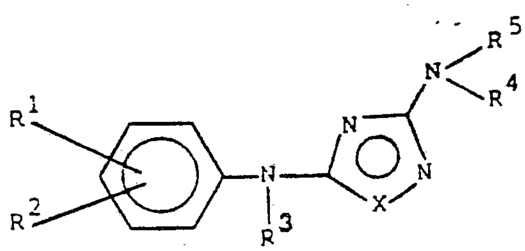

(I)

in which

$R^1$ is a hydrogen or halogen atom or an alkyl group,

$R^2$ is a halogen atom or an alkyl group,

$R^3$ is a hydrogen atom, an acyl group or a saturated or unsaturated alkyl group,

$R^4$ and $R^5$ which can be identical or different are a hydrogen atom or an alkyl group, it being possible for all the alkyl or acyl groups to be branched or unbranched, and each of them containing 1 to 4 carbon atoms, and

X is oxygen or sulphur, with the exception of 3-amino-5-(4-methyl-phenylamino)-1,2,4-oxadiazole, 3-amino-5-p-bromoanilino-1,2,4-thiadiazole and 3-amino-5-(p-tolyl-amino)-1,2,4-thiadiazole.

2. 3,5-Diamino-1,2,4-oxadiazoles of the general formula I according to Claim 1, in which —X— is —O— and $R^1$, $R^2$, $R^3$, $R^4$ and $R^5$ have the meaning given, at least one of the remaining substituents $R^2$, $R^3$, $R^4$ and $R^5$ being other than hydrogen if $R^1$ is a 4-methyl group.

3. 3-Amino-5-(2,6-dichlorophenylamino)-1,2,4-oxadiazole according to Claim 1.

4. 3,5-Diamino-1,2,4-thiadiazoles according to Claim 1.

5. Process for the preparation of the 1,2,4-oxadiazoles according to the general formula I according to Claim 1, in which X is an oxygen atom, $R^1$, $R^2$, $R^4$ and $R^5$ have the meaning given above and $R^3$ is a hydrogen atom, charcterised in that isocyanide dichlorides of the general formula II

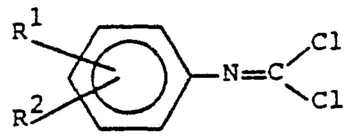

(II)

in which $R^1$ and $R^2$ have the meaning above, are cyclised with a hydroxyguanidine of the general formula III

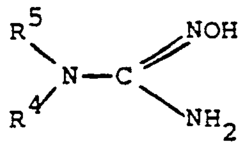

(III)

in which $R^4$ and $R^5$ have the meaning given above, in the presence of a base to give the 3,5-diamino-1,2,4-oxadiazoles according to the invention.

6. Process for the preparation of the 1,2,4-thiadiazoles according to the general formula I according to Claim 1, in which X is a sulphur atom and $R^1$, $R^2$, $R^4$ and R5 have the meaning given above and $R^3$ is a hydrogen atom, characterised in that amidinothioureas of the general formula IV

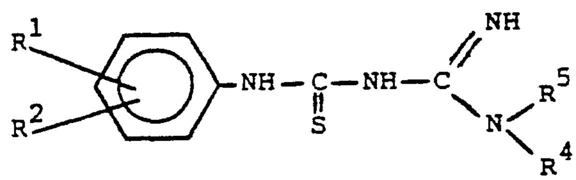

(IV)

in which $R^1$, $R^2$, $R^4$ and $R^5$ have the meaning given above, are oxidatively cyclised to give the 3,5-diamino-1,2,4-thiadiazoles.

7. Process for the preparation of compounds of the general formula I according to Claim 1, in which $R^3$ is an acyl group and $R^1$, $R^2$, $R^4$, $R^5$ and X have the meaning given, characterised in that compounds of the general formula I, in which $R^3$ is a hydrogen atom, are reacted with acyl halides or acid anhydrides.

8. Process for the preparation of compounds of the general formula I according to Claim 1, in which $R^3$ is an alkyl group and $R^1$, $R^2$, $R^4$, $R^5$ and X have the meaning given, characterised in that compounds of the general formula I, in which $R^3$ is a hydrogen atom, are reacted with alkyl halides.

9. Pharmaceutical preparation, characterised in that it contains one or more of the compounds of the general formula I according to Claim 1, or acceptable salts thereof and, if appropriate, conventional excipients and/or extenders.

10. Compounds of the general formula I according to Claim 1 for application in cases of hypertension.

**Claims** (for the contracting state AT)

1. Process for the preparation of substituted 3,5-diamino-1,2,4-oxadiazoles and 3,5-diamino-1,2,4-thiadiazoles of the general formula I

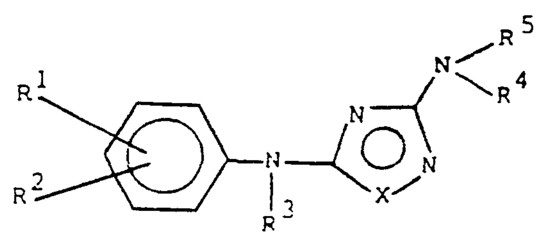

(I)

in which

$R^1$ is a hydrogen or halogen atom or an alkyl group,

$R^2$ is a halogen atom or an alkyl group,

$R^3$ is a hydrogen atom, an acyl group or a saturated or unsaturated alkyl group,

$R^4$ and $R^5$ which can be identical or different are a hydrogen atom or an alkyl group, it being possible for all the alkyl or acyl groups to be branched or unbranched, and each of them containing 1 to 4 carbon atoms, and

X is oxygen or sulphur, with the exception of 3-amino-5-(4-methyl-phenylamino)-1,2,4-oxadiazole, 3-amino-5-p-bromoanilino-1,2,4-thiadiazole and 3-amino-5-(p-tolyl-amino)-1,2,4-thiadiazole, characterised in that

a) for the preparation of the 1,2,4-oxadiazoles of the general formula I, in which X is an oxygen atom, $R^1$, $R^2$, $R^4$ and $R^5$ have the meaning given above and $R^3$ is a hydrogen atom, isocyanide dichlorides of the general formula II

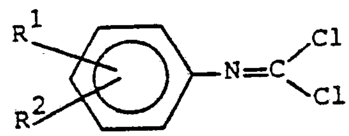

(II)

in which $R^1$ and $R^2$ have the meaning given above, are cyclised with a hydroxyguanidine of the general formula III

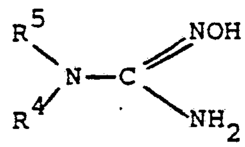

(III)

in which $R^4$ and $R^5$ have the meaning given above, in the presence of a base to give the 3,5-diamino-1,2,4-oxadiazoles according to the invention,

b) for the preparation of the 1,2,4-thiadiazoles according to the general formula 1, in which X is a sulphur atom and $R^1$, $R^2$, $R^4$ and $R^5$ have the meaning given above and $R^3$ is a hydrogen atom, amidinothioureas of the general formula IV

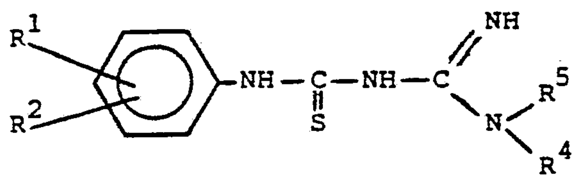

(IV)

in which $R^1$, $R^2$, $R^4$ and $R^5$ have the meaning given above, are oxidatively cyclised to give the 3,5-diamino-1,2,4-thiadiazoles,

c) for the preparation of compounds of the general formula I, in which $R^3$ is an acyl group and $R^1$, $R^2$, $R^4$, $R^5$ and X have the meaning given, compounds of the general formula I in which $R^3$ is a hydrogen atom, are reacted with acyl halides or acid anhydrides, and

d) for the preparation of compounds of the general formula I, in which $R^3$ is an alkyl group and $R^1$, $R^2$, $R^4$, $R^5$ and X have the meaning given, compounds of the general formula I, in which $R^3$ is a hydrogen atom, are reacted with alkyl halides.

2. Process according to Claim 1 for the preparation of 3-amino-5-(2,6-dichlorophenylamino)-1,2,4-oxadiazole, characterised in that 2,6-dichlorophenylisocyanide dichloride is cyclised with hydroxyguanidine.

**Revendications** (pour les Etats contractants : BE, CH, DE, FR, GB, IT, LI, NL, SE)

1. 3,5-Diamino-1,2,4-oxadiazoles et 3,5-diamino-1,2,4-thiadiazoles substitués de la formule générale I :

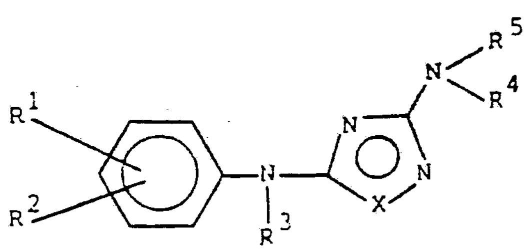

(I)

où

$R^1$ représente un atome d'hydrogène ou d'halogène ou un radical alcoyle,

$R^2$ représente un atome d'halogène ou un radical alcoyle,

$R^3$ représente un atome d'hydrogène, un radical acyle ou un radical alcoyle saturé ou insaturé,

$R^4$ et $R^5$, qui peuvent être identiques ou différents, représentent un atome d'hydrogène ou un radical alcoyle, les divers radicaux alcoyle ou acyle pouvant être ramifiés ou non ramifiés et compter chacun 1 à 4 atomes de carbone, et

X représente un atome d'oxygène ou de soufre, à l'exception

du 3-amino-5-p-bromoanilino-1,2,4-thiadiazole,

du 3-amino-5-(p-tolylamino)-1,2,4-thiadiazole, et

du 3-amino-5-(4-méthylphénylamino)-1,2,4-oxadiazole.

2. 3,5-Diamino-1,2,4-oxadiazoles de la formule générale I suivant la revendication 1, où X représente O et $R^1$, $R^2$, $R^3$, $R^4$ et $R^5$ ont les significations attribuées ci-dessus, étant entendu que si $R^1$ représente un radical 4-méthyle, au moins un des substituants $R^2$, $R^3$, $R^4$ et $R^5$ restants n'est pas un atome d'hydrogène.

3. Le 3-amino-5-(2,6-dichlorophénylamino)-1,2,4-oxadiazole suivant la revendication 1.

4. 3,5-Diamino-1,2,4-thiadiazoles suivant la revendication 1.

5. Procédé de préparation des 1,2,4-oxadiazoles de la formule générale I suivant la revendication 1, où X représente un atome d'oxygène, $R^1$, $R^2$, $R^4$ et $R^5$ ont les significations attribuées ci-dessus et $R^3$ représente un atome d'hydrogène, caractérisé en ce qu'on cyclise des dichlorures d'isocyanures de la formule générale II :

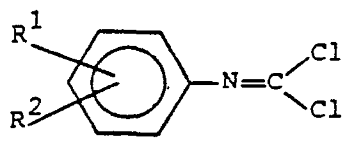

(II)

où $R^1$ et $R^2$ ont les significations attribuées ci-dessus,

avec une hydroxyguanidine de la formule générale III :

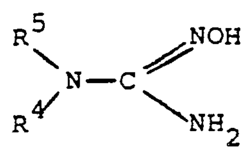

(III)

où $R^4$ et $R^5$ ont les significations attribuées ci-dessus, en présence d'une base en les 3,5-diamino-1,2,4-oxadiazoles conformes à l'invention.

6. Procédé de préparation des 1,2,4-thiadiazoles de la formule générale I suivant la revendication 1, où X représente un atome de soufre, $R^1$, $R^2$, $R^4$ et $R^5$ ont les significations attribuées ci-dessus et $R^3$ représente un atome d'hydrogène, caractérisé en ce qu'on cyclise des amidinothiourées de la formule générale IV :

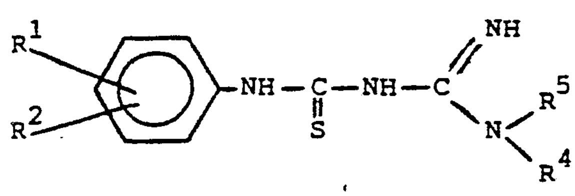

(IV)

où $R^1$, $R^2$, $R^4$ et $R^5$ ont les significations attribuées ci-dessus, par oxydation en les 3,5-diamino-1,2,4-thiadiazoles.

7. Procédé de préparation de composés de la formule générale I suivant la revendication 1, où $R^3$ représente un radical acyle et $R^1$, $R^2$, $R^4$, $R^5$ et X ont les significations attribuées ci-dessus, caractérisé en ce qu'on fait réagir des composés de formule générale I où $R^3$ représente un atome d'hydrogène avec des halogénures d'acyle ou anhydrides d'acides.

8. Procédé de préparation de composés de la formule générale I suivant la revendication 1, où $R^3$ représente un radical alcoyle et $R^1$, $R^2$, $R^3$, $R^5$ et X ont les significations attribuées ci-dessus, caractérisé en ce qu'on fait réagir des composés de formule générale I où $R^3$ représente un atome d'hydrogène avec des halogénures d'alcoyle.

9. Préparation pharmaceutique, caractérisée en ce qu'elle contient un ou plusieurs composés de la formule générale I suivant la revendication 1, ou leurs sels compatibles et éventuellement des excipients et/ou diluants habituels.

10. Composé de la formule générale I suivant la revendication 1 à administrer en cas d'hypertension.

**Revendications** (pour l'Etat contractant AT)

1. Procédé de préparation de 3,5-diamino-1,2,4-oxadiazoles et 3,5-diamino-1,2,4-thiadiazoles substitués de la formule générale I :

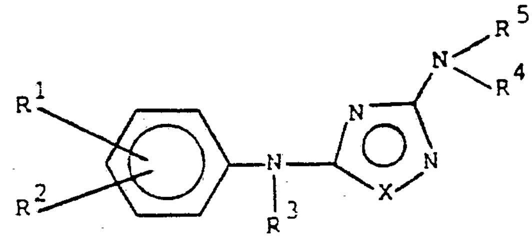

(I)

où

$R^1$ représente un atome d'hydrogène ou d'halogène ou un radical alcoyle,

$R^2$ représente un atome d'halogène ou un radical alcoyle,

$R^3$ représente un atome d'hydrogène, un radical acyle ou un radical alcoyle saturé ou insaturé,

$R^4$ et $R^5$, qui peuvent être identiques ou différents, représentent un atome d'hydrogène ou un radical alcoyle, les divers radicaux alcoyle ou acyle pouvant être ramifiés ou non ramifiés et compter chacun 1 à 4 atomes de carbone, et

X représente un atome d'oxygène ou de soufre, à l'exception
du 3-amino-5-p-bromoanilino-1,2,4-thiadiazole,
du 3-amino-5-(p-tolylamino)-1,2,4-thiadiazole, et
du 3-amino-5-(4-méthylphénylamino)-1,2,4-oxadiazole,
caractérisé en ce que

a) pour la préparation des 1,2,4-oxadiazoles de la formule générale I, où X représente un atome d'oxygène, $R^1$, $R^2$, $R^4$ et $R^5$ ont les significations attribuées ci-dessus et $R^3$ représente un atome d'hydrogène, on cyclise des dichlorures d'isocyanures substitués de la formule générale II :

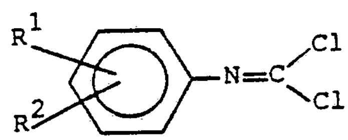

(II)

où $R^1$ et $R^2$ ont les significations attribuées ci-dessus, avec une hydroxyguanidine de la formule générale III :

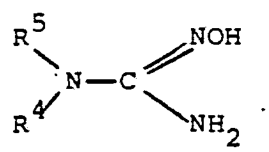

(III)

où $R^4$ et $R^5$ ont les significations attribuées ci-dessus, en présence d'une base, en les 3,5-diamino-1,2,4-oxadiazoles conformes à l'invention,

b) pour la préparation des 1,2,4-thiadiazoles de la formule générale I, où X représente un atome de soufre, $R^1$, $R^2$, $R^4$ et $R^5$ ont les significations attribuées ci-dessus, et $R^3$ représente un atome d'hydrogène, on cyclise des amidinothiourées de la formule générale IV :

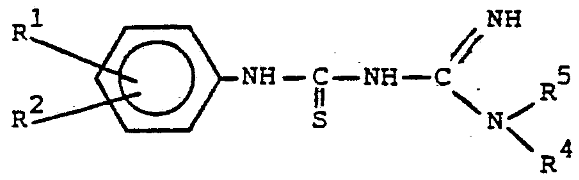

(IV)

où $R^1$, $R^2$, $R^4$ et $R^5$ ont les significations attribuées ci-dessus, par oxydation en les 3,5-diamino-1,2,4-thiadiazoles,

c) pour la préparation de composés de la formule générale I, où $R^3$ représente un radical acyle et $R^1$, $R^2$, $R^4$ et $R^5$ et X ont les significations attribuées ci-dessus, on fait réagir des composés de la formule générale I où $R^3$ représente un atome d'hydrogène avec des halogénures d'acyle ou anhydrides d'acides, et

d) pour la préparation de composés de la formule générale I, où $R^3$ représente un radical alcoyle et

$R^1$, $R^2$, $R^4$ et $R^5$ et X ont les significations attribuées ci-dessus, on fait réagir des composés de la formule I où $R^3$ représente un atome d'hydrogène avec des halogénures d'alcoyle.

2. Procédé suivant la revendication 1 de préparation du 3-amino-5-(2,6-dichlorophénylamino)-1,2,4-oxadiazole, caractérisé en ce qu'on cyclise le dichlorure d'isocyanure de 2,6-dichlorophényle avec l'hydroxyguanidine.